# EUROPEAN PATENT SPECIFICATION

(11) **EP 4 082 453 B1**
(45) Date of publication and mention of the grant of the patent: **11.06.2025**
(21) Application number: 22169866.5
(22) Date of filing: 25.04.2022
(51) Int. Cl.: A61B 17/15, A61B 17/17, A61B 34/10, A61C 8/00, A61B 90/00

(54) **SURGICAL DEVICE WITH PATIENT-SPECIFIC FIBULA MALLEOLUS CAP**
CHIRURGISCHE VORRICHTUNG MIT PATIENTENSPEZIFISCHER WADENBEINKNÖCHELKAPPE
DISPOSITIF CHIRURGICAL DOTÉ D'UNE COIFFE DE MALLÉOLE PÉRONIÈRE SPÉCIFIQUE AU PATIENT

(30) Priority: 26.04.2021 US 202163179685 P
(43) Date of publication of application: 02.11.2022
(73) Proprietor: Versitech Limited, Hong Kong (CN)
(72) Inventor: SU, Yuxiong, Sai Ying Pun (HK); PU, Jingya, Sai Ying Pun (HK)
(74) Representative: HGF

(56) References cited:
- EP-A1- 3 195 816
- US-A1- 2012 239 045
- US-A1- 2013 296 872
- US-A1- 2016 089 153
- US-A1- 2018 085 133
- SCHEPERS RUTGER H ET AL: "Accuracy of fibula reconstruction using patient-specific CAD/CAM reconstruction plates and dental implants: A new modality for functional reconstruction of mandibular defects", JOURNAL OF CRANIO-MAXILLO-FACIAL SURGERY, CHURCHILL LIVINGSTONE, GB, vol. 43, no. 5, 27 March 2015 (2015-03-27), pages 649 - 657, XP029611149, ISSN: 1010-5182, DOI: 10.1016/J.JCMS.2015.03.015

## Description

### FIELD OF THE INVENTION

This invention is generally directed **to** surgical devices for repair and reconstruction of head and neck defects.

### BACKGROUND OF THE INVENTION

The reconstruction of mandible defects using a free fibula graft was first described by Hidalgo in 1989 and is nowadays an established method in cranio-maxillofacial (CMF) surgery (Hidalgo, Plast. Reconstr. Surg.. 84:71-79 (1989)). The fibula bone is removed (the flap) along with two or three blood vessels, one of which supplies blood to the flap (the artery) and one or two of which drains blood from the flap (the vein). Once the bone is raised it is transferred to the head and neck region and secured in place with plates and screws. The blood vessels supplying and draining the flap are then joined to blood vessels in the neck under a microscope. These blood vessels then keep the flap alive while it heals into its new place. Initially, the procedure was performed freehand, and the surgeons' experience, routine, and skills were essential factors that potentially influenced the outcome, since the shaping of the fibula, as well as the bending of the plates, had to be performed intraoperatively.

In the years that followed, pre-bent reconstruction plates, electron beam melting manufactured reconstruction plates, prefabricated templates, and osteotomy guides (Weitz, et al., J. Cranio-Maxillofac. Surg., 46:1975-1978 (2018)) were also described and applied.

When computers became a part of everyday surgical practice, jaw reconstructions started to be performed using virtual surgical planning (VSP), which is now state of the art in many centers. Individual cutting guides and preformed reconstruction plates have been produced on the basis of digital 3D data, since then to assist the surgeon during the operation to perform osteotomies at the correct angle and in the right place, as well as to reconstruct the shape and position of the mandible according to the planned parameters. On the one hand, this improves surgical workflow in the operating theater, and on the other hand leads to better results compared to the freehanded method, especially in complex cases. In addition to the advantages of better surgical preparation and a reduction in the operation time, this method has disadvantages, such as dependence on industry and loss of time due to outsourced planning, production, and shipping of the template and the resulting high costs. Subsequently, template-guided surgery remains more expensive than freehand surgery (Meyer et al., J. Clin. Med., 9,4119:1-14 (2020)).

Computer-assisted surgery (CAS) and 3D printing have revolutionized head and neck reconstruction. A set of serial studies on CAS and 3D printing facilitated a paradigm shift in jaw reconstruction, leading to a new era of "digitalization and precision surgery" *(*Pu et al., Oral Oncol.;111:104914 (2020); Yang et al., Oral Oncol., 78:31-6 (2018); and Yang et al., Ann Surg Oncol., 28:363-375 (2021)). Using CAS, surgeons make virtual plans on a computer that will be executed in the operating room to guide the harvesting and placement of bone segments to repair defects. Bone segments can be finely navigated for optimal restoration of the original skeleton.

However, even with the most recent technology, the error of jaw reconstruction between the pre-operative planning and the surgical outcome is noticeable.

Fibula free flap is the most commonly used reconstruction method for bony defect of jaws. Although fibula looks like a uniformly shaped long bone, the geometric shape of its cross-sectional anatomy actually differs a lot along its axis. Thus, the accurate positioning of the fibula harvest guide is of premier importance to achieve the desired VSP in real surgery. However, it is well known that the fit for fibula harvest guides is less than ideal. In fact, in order to avoid undercuts which might prevent adaptation of guide to the fibula surface and protect the vessel pedicle on the medial surface of fibula, only the geometry of the lateral surface can be used as a reference when designing the fibula harvest guide. This leads sliding and rotational errors when positioning the fibula harvesting guide. The traditional approach is to measure the distance from the lateral malleolus to locate the fibula harvesting guide. However, this approach brings inaccuracy. The lateral malleolus is a rounded three-dimensional structure. The surgeon might take different reference points in the virtual surgical planning. Besides, the movable soft tissue will prevent the correct positioning of the ruler when the measurement is done intraoperatively. In addition to the longitudinal sliding error, the harvest guide may also rotate surrounding the long axis of the fibula due to the relatively rounded shape of the fibula in its cross-section especially when covered with periosteum and a thin cuff of muscle.
Schepers et al. Journal of Cranio-Maxillo-Facial Surgery; 43 (2015), 649-657 describes a study to analyze the accuracy of mandibular reconstruction using patient-specific computer-aided designed and a computer-aided manufactured (CAD/CAM) reconstruction plates as a guide to place fibula grafts and dental implants. The study discloses a fibula drilling and resection guide connected to a malleolus cap by a connection bar.

There remains a need to improve reconstruction accuracy to facilitate the accurate functional jaw reconstruction and dental rehabilitation.

Therefore, it is the object of the present invention to provide surgical devices that reduce sliding and rotational errors of the fibula harvest guide to increase the accuracy of jaw reconstruction.

### SUMMARY OF THE INVENTION

The invention relates to a surgical device as defined in the appended claims.

Described are fibula malleolus caps and surgical devices containing fibula malleolus caps. According to the invention, the fibula malleolus caps in the devices are connected to fibula osteotomy guides via connecting bars. The fibula malleolus caps are patient-specific malleolus caps.

The malleolus caps typically include an inner cap surface and an outer cap surface, the outer cap surface contacting one end of the connecting bar. The inner cap surface may have a morphology modelled to fit the surface morphology of the patient's lateral malleolus.

Generally, the longitudinal axes of the malleolus cap, the connecting bar, and the osteotomy guide are offset relative to one another in the device. The connecting bar may contact the malleolus cap and the osteotomy guide via connecting rods. The inner surface of the malleolus cap in the device may be relieved relative to the inner surface of the osteotomy guide to allow for skin covering the bony malleolus. The relief in the inner surface of the malleolus cap relative to the inner surface of the osteotomy guide may be between about 0.1 mm and about 2 mm, between about 0.1 mm and about 1 mm, or about 0.5 mm for the skin covering the bony malleolus.

Typically, the malleolus cap, the osteotomy guide, and the connecting bar of the device are formed from biocompatible, autoclavable resin, polymer, metal, or metal alloy. The surgical device may be made by any suitable method, such as by stereolithography, soft lithography, laser machining, laser cutting, micromachining, micromilling, curing, bonding, three-dimensional printing, molding, micromolding, and/or setting.

The surgical device is typically used in reconstruction surgery of head and neck bone defects, such as in jaw reconstruction surgery. The surgical device may be used in jaw reconstruction surgery with simultaneous (during the same surgery) dental implant placement.

Also described are methods for jaw reconstruction using the malleolus cap and the devices with the malleolus cap. These methods obtain the fibula free flap with minimal sliding and/or rotational errors of the osteotomy guide. The methods also provide accurate placement of the fibula free flap in position in the jaw, including accurate placing of dental implants in at least a portion of reconstructed jaw. Typically, the devices provide minimal rotational errors of the osteotomy guide when angle deviation, measured as angulation between real osteotomy plane and planned osteotomy plane, is between 0 degrees and about 15 degrees. The devices provide minimal sliding errors of the osteotomy guide when axial deviation, measured as the distance between intersection points generated by the real and planned osteotomy planes, is between 0 mm and about 8 mm.

The devices also provide accurate placement of the implant in position in the jaw. The use of the developed fibula malleolus cap significantly increased the accuracy of the fibula osteotomy thus making the dental prosthetic rehabilitation with simultaneous dental implants more precise, approaching the similar accuracy level of the guided implant surgeries in native maxilla and mandible.

Typically, the devices provide accurate placement of the implant in position in the jaw when implant platform deviation, measured as a difference between center point of implant platform in the virtual surgical plan and center point of implant platform in the actual implant, is between about 0 mm and about 3 mm. The devices may also provide accurate placement of the implant in position in the jaw when the apex deviation, measured as a difference between apex of the implant in the virtual surgical plan and apex of actual implant placed, is between about 0 mm and about 3.5 mm. The devices may also provide accurate placement of the implant in position in the jaw when the angle deviation, measured as angle deviation of long axes of actual implant position and implant position in the virtual surgical plan, is between about 0 degrees and about 8 degrees.

### BRIEF DESCRIPTION OF THE DRAWINGS

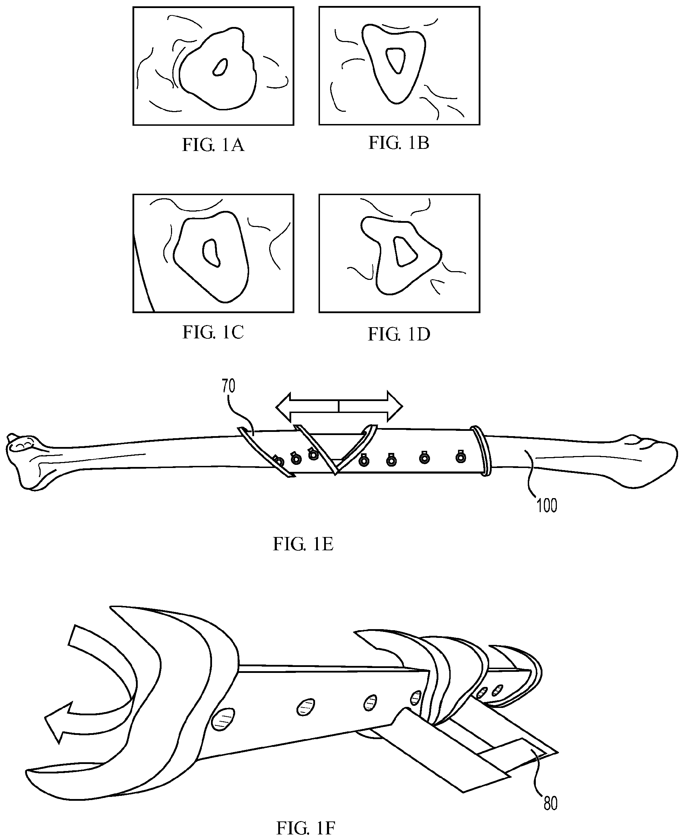

**Figures 1A-1D** are cross-sectional CT images of a fibula showing different geometirc shapes along the fibula length - oval **(****Figure 1A****),** triangular **(****Figure 1B****),** quadrilateral **(****Figure 1C****),** and pentagonal **(****Figure 1D. Figure 1E** is a diagram showing the sliding error in the axial direction (thick bars) of the osteotomy guide **70** over fibula **100.** **Figure 1F** is a diagram showing the rotational error in cross-sectional direction, of the osteotomy guide **80.**

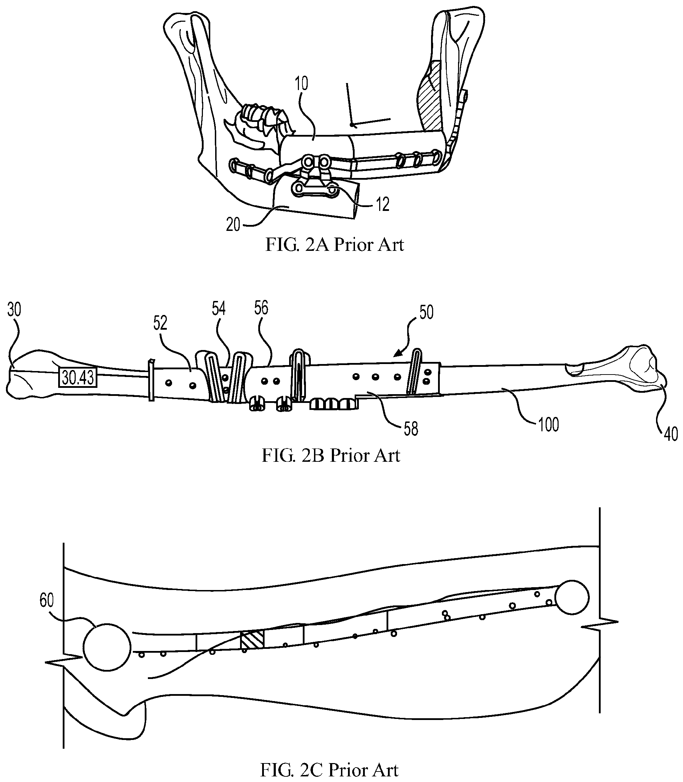

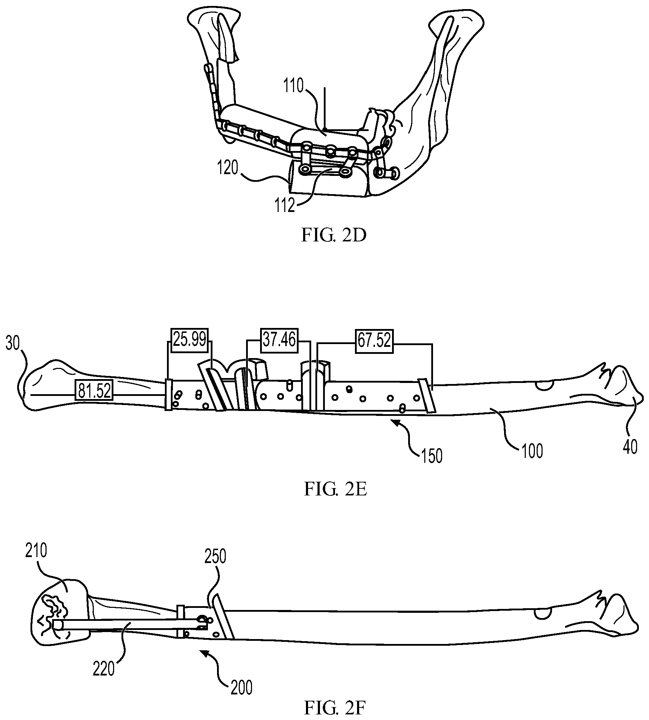

**Figures 2A-2C** are diagrams showing the steps in mandible reconstruction of the prior art during which the fibula free flap is harvested without the use of a malleolus cap. **Figures 2D-2F** are diagrams showing the steps in mandible reconstruction using fibula free flap conducted with the use of a malleolus cap.

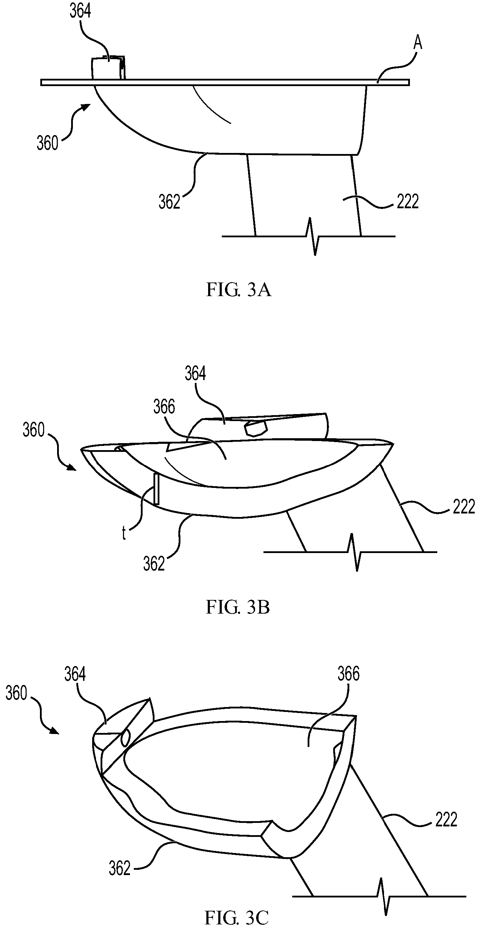

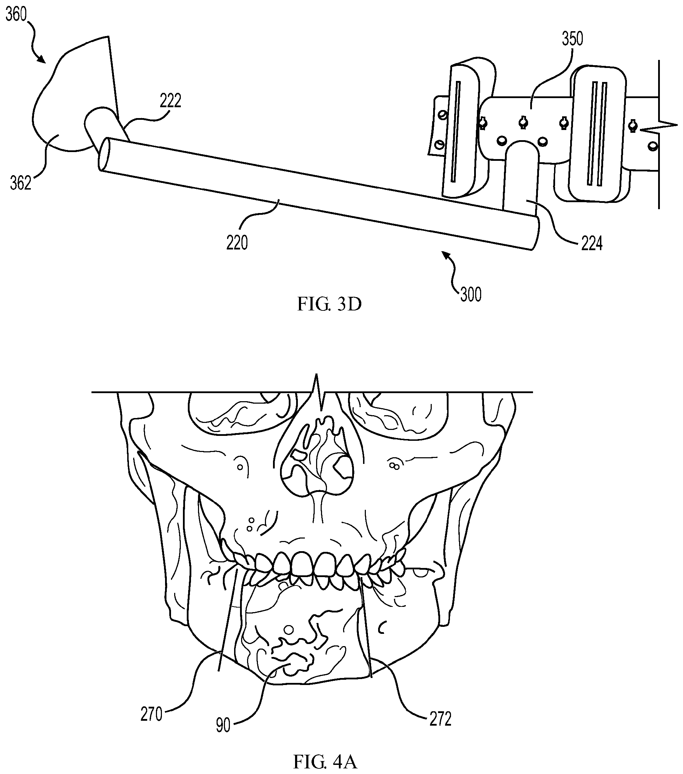

**Figures 3A-3C** are diagrams showing an exemplary malleolus cap **360** from side view **(****Figures 3A and 3B****),** and bottom perspective view **(****Figure 3C****).** **Figure 3D** **is** a diagram showing top perspective view of the surgical device **300.**

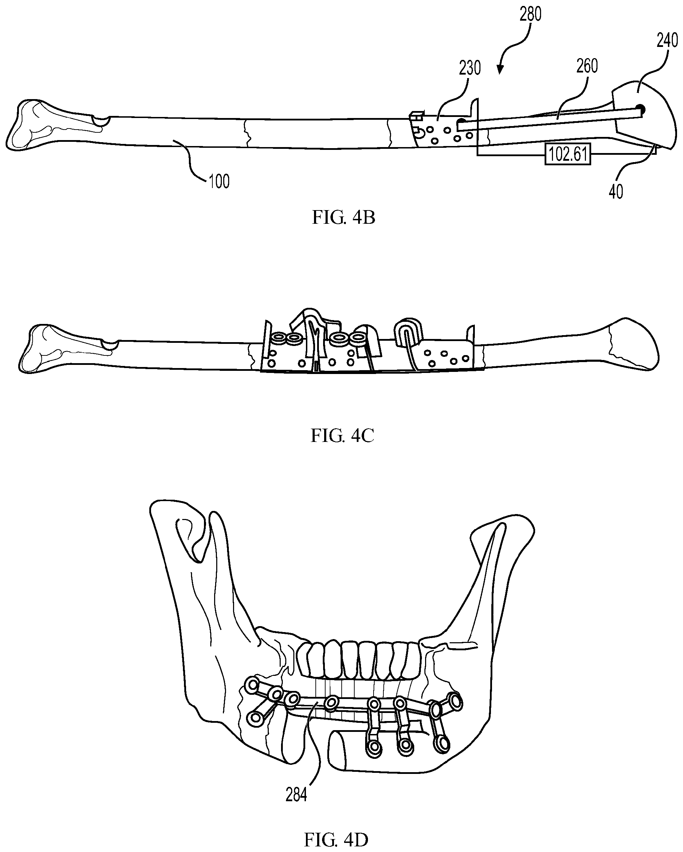

**Figures 4A-4D** are diagrams showing mandible reconstruction using resection guide locations (Figure 4A), a fibula free flap harvesting guide with malleolus cap **(****Figure 4B****),** the osteotomy guide designs **(****Figure 4C****)** and 3D-printed patient-specific surgical plate designed to fix bone segments with dental implants **(****Figure 4D****).**

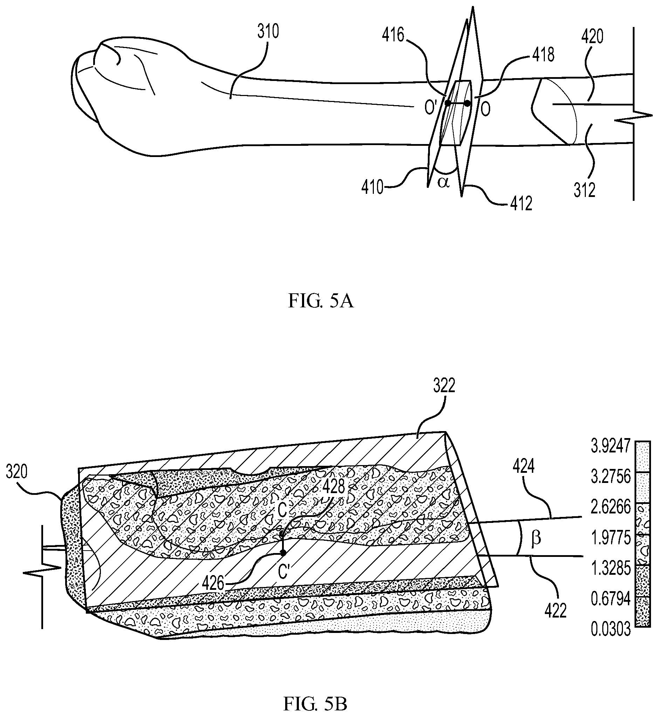

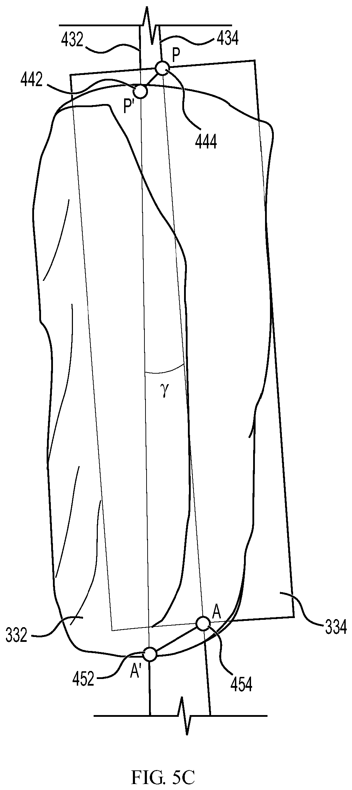

**Figures 5A-5C** are diagrams showing references used in accuracy analyses. **Figure 5A** shows fibula distal osteotomy accuracy analyses. The distal end of fibula after harvest **310** is aligned with fibula in pre-operative virtual surgical planning **420.** O (point **418)** is center of the planned osteotomy plane **412.** O' (point **416)** is center of the actual osteotomy plane **410.** O-O': Axial deviation (mm) of the osteotomy plane. **α**: Angle deviation (degrees) of the osteotomy plane. **Figure 5B** shows reconstruction segment accuracy analyses. C (point **428)** is center of the planned fibula segment. C' (point **426)** is center of the actual fibula segment. C-C': Center point deviation (mm) of the fibula segment. **β**: Angle deviation of the fibula segment. Absolute distance deviation represented in the color map. **Figure 5C** shows implant accuracy analyses. Cylinder **334:** Implant position in the virtual surgical plan. Cylinder **332:** Actual implant position in the postoperative CT scan. P (point **444)** is the center point of implant platform in the virtual surgical plan. P'(point **442)** is the center point of implant platform in the actual implant. A (point **454)** is the apex of the implant in the virtual surgical plan. A' (point **452)** is the apex of actual implant placed. P-P': Deviation in implant platform position (mm). A-A': Deviation in implant apex position (mm). **γ**: Angle deviation of long axes **432** and **434** of implants.

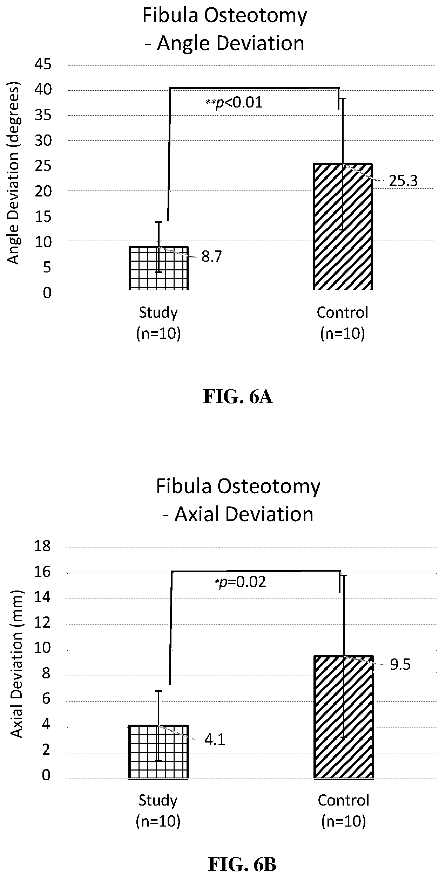

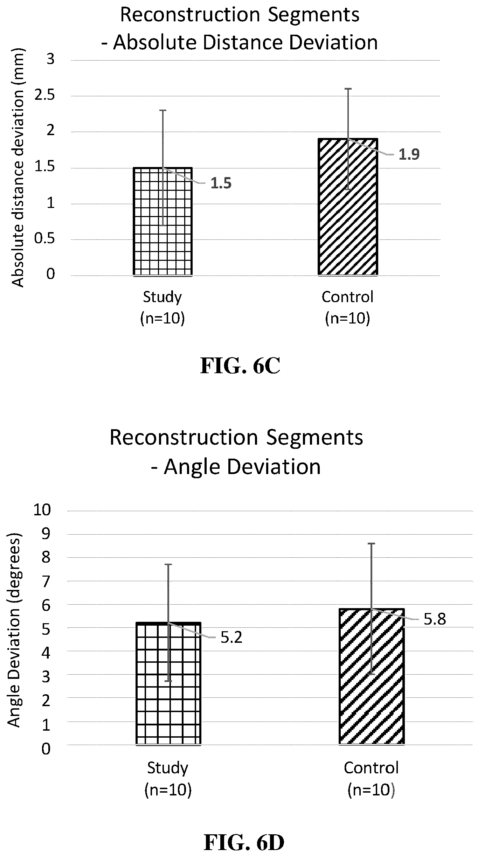

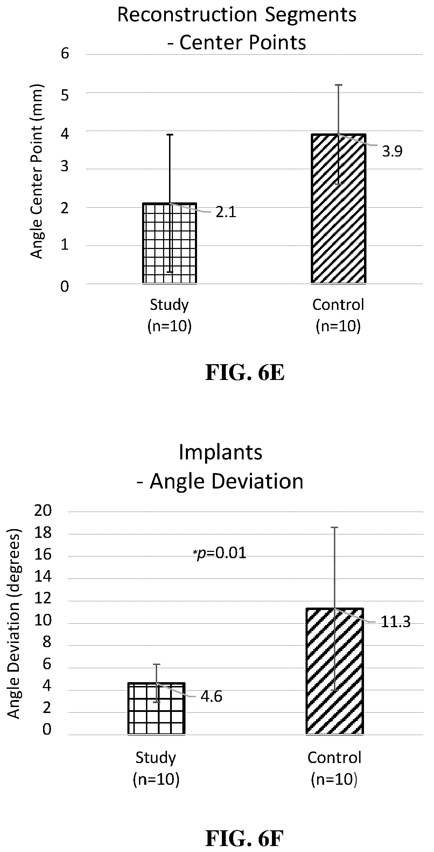

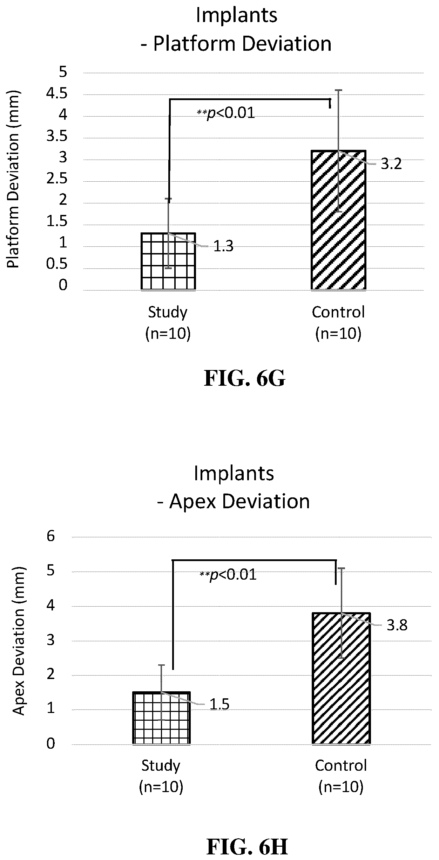

**Figures 6A-6H** are graphs showing the results from the accuracy analyses between the study group (n=10) and the control group (n=10). Figures **6A** and **6B** show angle deviation (degrees, **Figure 6A****)** and axial deviation (mm, **Figure 6B****)** for fibula distal osteotomy accuracy analyses between the control and the study groups. **Figures 6C-6E** show absolute distance deviation (mm, **Figure 6C****),** angle deviation (degrees, **Figure 6D****),** and center points (mm, **Figure 6E****)** for reconstruction segment accuracy analyses between the control and the study groups. **Figures 6F-6H** show angle deviation (degrees, **Figure 6F****),** platform deviation (mm, **Figures 6G****),** and apex deviation (mm, **Figure 6H****)** for implant accuracy analyses between the control and the study groups. All p-values lower than 0.05 and 0.01 are indicated with an asterisk (*) and double asterisks (**), respectively.

### DETAILED DESCRIPTION OF THE INVENTION

### I. Definitions

As used herein, the term "3D printing" refers to the successive layer overlapping manufacturing of customized products based on computer-designed digital files. "Additive manufacturing" or "3D printing" as used herein refers to a process of making a three-dimensional solid object of virtually any shape from a digital model. 3D printing is achieved using an additive process, where successive layers of material are laid down in different shapes or thicknesses. The material may be jetted or extruded through a nozzle and solidified into a desired shape. The shape can be controlled in the x, y and z directions.

As used herein, the term "patient-specific", in the context of a cap, guide, or a device, refers to a surface morphology or contours modelled according to the morphology of an anatomical site of the intended patient. The surface morphology or contour may be modeled to fit over an anatomical site of the patient without substantial gaps between the surface morphology or contour and the anatomical site.

As used herein, the term "substantial" generally refers to a comparative degree, such as at least about 90%, preferably about 95%, or more preferably about 98% or more for substantial similarity, and less than 10%, preferably less than 5%, or less than 2% for substantial difference.

As used herein, the terms "subject," "individual" or "patient" refer to a human or a non-human mammal. A subject may be a non-human primate, domestic animal, wild animal, farm animal, or a laboratory animal. For example, the subject may be a dog, cat, goat, horse, pig, mouse, rabbit, rat, or the like. The subject may be a human. The subject may be healthy or suffering from or susceptible to a disease, disorder or condition. A patient refers to a subject afflicted with a disease or disorder. The term "patient" includes human and veterinary subjects.

A "control" sample or value refers to a sample or value that serves as a reference, usually a known reference, for the test sample or value. For example, a test sample can be taken from a test subject, and a control sample can be taken from a control subject, such as from a known normal (non-disease) individual, or diseased individual that did not undergo the test treatment. A control can also represent an average value gathered from a population of similar individuals, e.g., disease patients or healthy individuals with a similar medical background, same age, weight, etc. One of skill will recognize that controls can be designed for assessment of any number of parameters.

As used herein the terms "treatment" or "treating" refer to one or more surgical steps to replace an affected bone in head or neck and reduce or alleviate one or more symptoms of a disease. The effect of the surgical steps on the subject can be, but is not limited to, the cessation of a particular symptom of a condition, a reduction or prevention of the symptoms of a condition, a reduction in the severity of the condition, the complete ablation of the condition, a stabilization or delay of the development or progression of a particular event or characteristic, or minimization of the chances that a particular event or characteristic will occur.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other embodiments the values may range in value either above or below the stated value in a range of approx. +/- 5%; in other embodiments the values may range in value either above or below the stated value in a range of approx. +/- 2%; in other embodiments the values may range in value either above or below the stated value in a range of approx. +/- 1%.

### II. Malleolus Cap and Surgical Device

Described are malleolus caps for fibula flap harvesting. The caps are used in jaw reconstruction surgeries requiring fibula osteotomy, jaw reconstruction, and, if needed, simultaneous (during the same surgery) dental implant placement. A comparative study performed to evaluate the effectiveness of the malleolus cap and devices containing the malleolus cap in enhancing the accuracy of fibula osteotomy, jaw reconstruction, and simultaneous dental implant placement revealed significantly lower osteotomy errors and significantly higher jaw implant placement accuracy.

### A. Malleolus Caps

Typically, the malleolus cap is a device attachment designed to substantially fit the surface morphology of a lateral malleolus of a fibula. The malleolus cap typically has an outer surface in a form of a partial spherical shape, a partial oval shape, a convex shape, a raised mound shape, or a flat shape. The malleolus cap typically has an inner surface shaped to substantially fit the lateral malleolus of the fibula. The malleolus cap may have a thickness between about 1 mm and about 10 mm, between about 1 mm and about 8 mm, between about 1 mm and about 6 mm, or about 3 mm. The major diameter of the cap, measured from the outer most opposing points on the cap in a longitudinal plane may be between about 10 mm and about 100 mm, between about 10 mm and about 80 mm, or between about 10 mm and about 60 mm. The minor diameter of the cap, measured from the outer most opposing points on the cap in a transverse plane may be between about 5 mm and about 50 mm, between about 10 mm and about 40 mm, between about 10 mm and about 30 mm, or between about 10 mm and about 20 mm.

The inner surface of the malleolus cap may be shaped to substantially fit the surface morphology of a given patient's lateral malleolus. In this embodiment, the malleolus cap is a patient-specific malleolus cap and is designed to substantially fit the specific surface morphology of the patient's lateral malleolus.

In an alternative embodiment not conform to the claimed invention, the inner surface of the malleolus cap may be designed to fit lateral malleolus of an average size. In this embodiment, the dimensions and the inner surface of the malleolus caps may vary based on an average lateral malleolus size in the population and may be, for example, in three sizes, such as small, medium, and large.

The malleolus cap may be detachably or non-detachably connected to other device components to form a surgical device. Typically, a portion of the outer surface of malleolus cap contacts one or more connecting rods of one or more connecting bars. The contact may be temporary through a detachable connection, or permanent through a non-detachable connection. The detachable connection may be by snapping into the malleolus cap, screwing onto the malleolus cap, fitting into the malleolus cap, inserting into the malleolus cap, or other similar methods, the connecting rod(s) of the connecting bar. The non-detachable connection may be by sealing onto the malleolus cap, molding with the malleolus cap, gluing onto the malleolus cap, additive manufacturing with the malleolus cap, or other similar methods, the connecting rod(s) of the connecting bar.

### B. Devices with Malleolus Caps

Described are surgical devices containing a malleolus cap connected to an osteotomy guide via a connecting bar. Typically, the malleolus cap has a longitudinal axis (A) substantially parallel to the longitudinal axis of the connecting bar and the longitudinal axis of the osteotomy guide. Generally, the longitudinal axes of the malleolus cap, the connecting bar, and the osteotomy guide are parallel and are offset relative to one another. The longitudinal axes of the malleolus cap, the connecting bar, and the osteotomy guide are offset relative to one another by a distance between about 0.2 mm and 50 mm.

The inner surface of the cap may be relieved relative to the inner surface of the osteotomy guide to allow for skin covering the bony malleolus. The relief in the inner surface of the malleolus cap relative to the inner surface of the osteotomy guide may be between about 0.1 mm and about 2 mm, between about 0.1 mm and about 1 mm, or about 0.5 mm for the skin covering the bony malleolus.

### 1. Malleolus Cap

The malleolus cap is described in section **II.A.**

### 2. Osteotomy Guide

The device may include any suitable osteotomy guide for marking and/or cutting the fibula. Fibula cutting guides and fibula harvesting guides are generally known in the art (Meyer et al., J. Clin. Med., 9, 4119:1-14 (2020), Yang et al., Ann Surg Oncol.,28:363-375 (2021)). The guides may be universally sized to fit any fibula in a population. The guides may be sized to fit small, medium, and large fibula sizes in a population. The guides may be patient-specific. The patient-specific guides are designed and manufactured to fit the dimensions and morphology of a given patient's fibula. The guides may include holes for pre-determined locations of plate screws and/or implants.

The guides may be detachably or non-detachably connected to other device components to form a surgical device. Typically, a portion of the outer surface of the guide contacts one or more connecting rods of one or more connecting bars. The contact may be temporary through a detachable connection, or permanent through a non-detachable connection. The detachable connection may be by snapping into the guide, screwing onto the guide, fitting into the guide, inserting into the guide, or other similar methods, the connecting rod(s) of the connecting bar. The non-detachable connection may be by sealing onto the guide, molding with the guide, gluing onto the guide, additive manufacturing with the guide, or other similar methods, the connecting rod(s) of the connecting bar.

### 3. Connecting Bar

The malleolus cap and the guide are typically connected to a rigid connecting bar. The connecting bar may have a thickness between about 0.5 mm and about 50 mm, between about 0.5 mm and about 25 mm, or about 10 mm. The length of the longitudinal connecting bar typically varies based on the position of the guide relative to the lateral malleolus. In some forms, the connecting bars may be extendible. In some forms, the connecting bars may be non-extendible. For example, the connecting bar can have a length of between about 2 cm to about 70 cm, about 5 cm to about 70 cm, or about 10 cm to about 50 cm. All of these ranges can be the range of adjustment of the length of an extendible connecting bar.

Typically, the connecting bar includes at least two ends. The connecting bar may include one or more connecting rods contacting the outer surface of the malleolus cap at one end of the connecting bar. The length of the transverse connecting rods may be about 30 mm to give enough space for the flap elevated at the lower leg. The connecting bar may include one or more connecting rods contacting the outer surface of the guide at the other end of the connecting bar. The connecting rods of the connecting bar may contacts the outer surface of the malleolus cap and the outer surface of the osteotomy guide at an obtuse, right, or acute angle.

### 4. Exemplary Device and Cap for jaw reconstruction without dental implants

**Figures 2D-2F** are diagrams showing the steps in mandible reconstruction using fibula free flap conducted with the use of a malleolus cap. **Figure 2D** is a diagram of a virtual surgical planning requiring reconstructions of segments **110** and **120** obtained from the fibula of the same patient using fibula segmentation guide **150** positioned over the fibula **100** having fibula **head 30** and lateral malleolus **40 (****Figure 2E****). The** osteotomy guide **250 (****Figure 2F****)** is used to produce the segments **110** and **120 (****Figure 2D****)** from the fibula **100 (****Figure 2E****).** The segments **110** and **120** are secured in the jaw using patient-specific titanium plate **112 (****Figure 2D****).** The osteotomy guide **250** is attached to the malleolus cap **210** via the connecting bar **220** and connecting rods and forms the harvest device **200 (****Figure 2F****).**

By contrast, the prior art performs the jaw reconstruction surgery without the use of the malleolus cap. **Figure 2A** is a diagram of a virtual surgical planning requiring reconstructions of segments **10** and **20** with conventional surgery steps. The segments **10** and **20** are obtained from the fibula **100** of the patient using fibula segmentation guide **50** containing segments **52, 54, 56,** and **58 (****Figure 2B****).** The fibula **100** includes the fibula head **30** and the lateral malleolus **40.** The fibula segments **10** and **20** are placed in the jaw and secured in place with patient-specific titanium plate **12 (****Figure 2A****).** The conventional method uses the outer skin markings **60 (****Figure 2C****)** to position the segmentation and harvest guide.

### a. Exemplary Malleolus Cap

**Figures 3A-3D** are diagrams showing an exemplary malleolus cap **360** with the outer surface **362,** inner surface **366,** and a distal stopper **364** positioned at the tip of the malleolus cap, on the anterior edge of malleolus cap **360.** The inner surface **366** typically fits the shape of the individual fibula lateral malleolus. The cap is typically relieved from bone surface by 0.5 mm to fit on top of the skin. The distal stopper **364** typically controls the axial location of the harvest guide.

The malleolus cap **360** typically has a thickness **(t)** of about 3 mm if printed from resin. Transparent materials preferred for easy check of the fit of malleolus cap.

**Figure 3D** is a diagram of a surgical device **300** with a malleolus cap **360.** The malleolus cap **360** may detachably or non-detachably connected to an osteotomy guide **350** via a connecting bar **220.** The connecting bar **220** typically contacts the malleolus cap **360** and the osteotomy guide **350** via connecting rods **222** and **224** positioned at opposite ends of the connecting bar **220.** In the transverse cross section, the connecting rods **222** and **224** may have a diameter of about 10 mm, and a length of about 30 mm. The connecting rods **222** and **224** may be positioned at a posterior edge of the malleolus cap **360** and osteotomy guide **350** to give space for the skin flap during fibula harvesting. The connecting rods **222 and 224** typically contact the outer surface of the malleolus cap **360** and the osteotomy guide **350.**

The connecting bar **220** typically has a diameter of about 10 mm. The length of the connecting bar **220** may vary and depend on the location of osteotomy guide. The connecting bar may or may not be extendible.

### 5. Exemplary Device and Cap for jaw reconstruction with dental implants

**Figures 4A-4D** are diagrams showing mandible reconstruction using resection guides and fibula free flap harvested with malleolus cap and reconstructed. A 32-year-old male with ossifying fibroma **90** in the anterior mandible was operated on and received segmental mandibulectomy and reconstruction using fibula free flap harvested with lateral malleolus cap. Preoperative CT image indicated the destructive mass in the anterior mandible. The 3D- printed patient-specific surgical plate designed to fix bone segments with dental implants were fixed intra-orally in locations **270** and **272 (****Figure 4A****)** A computer plan of the harvest guide **230** with malleolus cap **240** and connecting bar **260** of the harvest device **280** positioned over fibula **100** is shown in **Figure 4B****.** The osteotomy guide designs are shown in **Figure 4C****.** The 3D-printed patient-specific surgical plate **284** was designed to fix bone segments with dental implants **(****Figure 4D****).** The bone-plate complex was transferred to repair the defect site between the locations **270** and **272.** Intraoral image showed the accurate position of implants as planned. Immediate loading of dental implants with fixed dental bridges was conducted. An excellent occlusal relationship was achieved.

### C. Materials for the Device and Cap

Suitable materials for forming the devices with malleolus caps, malleolus caps, guides and plates include biocompatible autoclavable resins, natural and synthetic polymers, and metals. The metals include grade 5 titanium, titanium alloys, nickel-titanium alloys, cobalt chromium alloys, stainless steel alloys, copper alloys, iron and/or ferrous alloys, nichrome, zinc and galvanized materials, tantalum, kanthal, or cupronickel.

The polymers typically are biocompatible and autoclavable, and include MED625FLX, MED610 and MED620 by Stratasys Ltd., United States, NextDent SG (Vertex Dental, The Netherlands), polydimethylsiloxane (PDMS), polysulfone (PSF), commercial pure titanium or its alloy, and other materials. PDMS is a versatile elastomer that is easy to mold, and PSF is a rigid, amber colored, machinable thermoplastic. Other suitable materials include biologically stable thermosetting and thermoforming polymers, including polyethylene, polypropylene, polyoxymethylene (POM) - also known as acetal, polyacetal, and polyformaldehyde, polymethylmethacrylate, polyurethane, polysulfones, polyetherimide, polyimide, ultra-high molecular weight polyethylene (UHMWPE), crosslinked UPMWPE and members of the polyaryletherketone (PAEK) family, including polyetheretherketone (PEEK), carbon-reinforced PEEK, and polyetherketoneketone (PEKK).

### III. Methods of Making the Devices and Caps

### A. Methods of Making

The devices and caps may be made using methods known in the art and include stereolithography, soft lithography, laser machining, laser cutting, micromachining, micromilling, curing, bonding, three-dimensional (3D) printing, molding, micromolding, and setting.

The malleolus cap, osteotomy guide, and the connecting bar may be made by any of the stereolithography, soft lithography, laser machining, laser cutting, micromachining, micromilling, curing, bonding, three-dimensional printing, molding, micromolding, and setting methods. The preferred methods of making the malleolus cap and osteotomy guide is by additive manufacturing, such as by three-dimensional printing.

Additional methods may include drilling screw holes in the osteotomy guide to provide location guides where the plate screws and/or implants will be placed on the flaps.

An exemplary method of making a malleolus cap and osteotomy guide includes the steps of:
1. pre-operative virtual surgical planning;
2. designing the malleolus cap and osteotomy guide;
3. 3D printing the malleolus cap and osteotomy guide; and
4. attaching the malleolus cap to the osteotomy guide via a connecting bar.

The connecting bar may be offset from the longitudinal axis of the malleolus cap and the osteotomy guide. The offset may be achieved by joining the connecting bar to the malleolus cap and the osteotomy guide via connecting rods. The joining may be by screwing, gluing, melting, or printing the combination of the connecting bar, the malleolus cap, and the osteotomy guide as one unit.

Typically, the step of pre-operative virtual surgical planning utilizes computer-assisted jaw reconstruction using the acquired and segmented CT data of patient's fibula and jaw to construct the 3D model of the donor fibula and the recipient jaw. The computer-assisted reconstruction allows to conduct virtual reconstructive surgeries, including virtual surgeries to simultaneously (during the same surgery) position dental implants.

Typically, the 3D models of the donor's fibula and the recipient jaw serve as a guide to design and form malleolus cap and osteotomy guide, including patient-specific malleolus cap and osteotomy guide. Preferred methods of forming the malleolus cap and the osteotomy guide include 3D printing.

### B. Forming Patient-Specific Malleolus Cap

Typically, the malleolus cap is designed to fit the lateral malleolus of the fibula. The malleolus cap is typically formed to have an inner surface that fits the surface morphology of patient's lateral malleolus. The surface morphology of patient's lateral malleolus may be obtained from the CT data of patient's fibula. The inner surface of the malleolus cap is then designed to have the fitting shape using computer-assisted pre-operative virtual surgical planning. The designed malleolus cap is then manufactured to have the inner surface morphology fitting the patient's lateral malleolus.

The malleolus cap may have a thickness between about 1 mm and about 10 mm, between about 1 mm and about 8 mm, between about 1 mm and about 6 mm, or about 3 mm. The lateral malleolus cap was designed as a 3 mm thick cap that fit the surface morphology of the specific patient. The inner surface of the cap may be relieved relative to the inner surface of the osteotomy guide to allow for skin covering the bony malleolus. The relief in the inner surface of the malleolus cap relative to the inner surface of the osteotomy guide may be between about 0.1 mm and about 2 mm, between about 0.1 mm and about 1 mm, or about 0.5 mm for the skin covering the bony malleolus.

The malleolus cap is typically connected to an osteotomy guide using one or more rigid connecting bars. The connecting bars may have a thickness between about 0.5 mm and about 50 mm, between about 0.5 mm and about 25 mm, or about 10 mm. In some forms, the connecting bars may be extendible. In some forms, the connecting bars may be non-extendible. For example, the connecting bar can have a length of between about 2 cm to about 70 cm, about 5 cm to about 70 cm, or about 10 cm to about 50 cm. All of these ranges can be the range of adjustment of the length of an extendible connecting bar.

The malleolus cap may be 3D-printed with ISO-certified bio-compatible autoclavable MED610 resin (Stratasys Ltd., United States) or NextDent SG (Vertex Dental, The Netherlands).

### IV. Methods of Using the Device and Cap

### A. Placing the Device and Cap

The malleolus cap is typically fitted onto the lateral malleolus of the patient with the manual pressure in a medial-posterior direction to locate the cap anterior-posteriorly. Then the cap is pushed superiorly until the distal stopper tightly engages the inferior end of the malleolus. When the cutting guide is accurately located, the osteotomy guide fits onto the fibula bone surface automatically. In this way, the location of the osteotomy guide is controlled in all three dimensions.

Simultaneous dental implants may be placed into the fibula before segmentation. Fibula flap segments are then transferred to repair the defect and are fixed to the remaining jaw with 3D-printed patient-specific titanium plates.

### B. Determining Accuracy in Jaw Reconstruction

To determine accuracy of the jaw reconstruction post-operatively, the spiral CT scans of the lower limbs and reconstructed jaws are typically acquired post-operatively. Based on the data of the CT scan, three-dimensional models of the distal end of the remaining fibula, reconstructed jaws, and simultaneous dental implants, if present, are built using computer-assisted programs, such as ProPlan CMF 2.0 software (Materialise, Leuven, Belgium). The 3D models are then imported into another computer program, such as to 3-Matic 13.0 (Materialise), for comparison of the corresponding items with the pre-operative plan.

For this comparison, reference points, apexes, and planes are established, as show in **Figures 5A-5C****.** The comparison of the reference points, apexes, and planes between the pre-operative plan and the post-operative 3D models is conducted to obtain the difference in position and angulation between the pre-operative plan and the post-operative 3D models.

### 1. Minimizing Harvesting Errors

### a. Minimizing Sliding Errors

To calculate the sliding errors, the intersection points are established on the long axis of the fibula. The intersection points are formed by the real and planned osteotomy planes intersecting the long axis of the fibula. The difference in distance between these points is the axial deviation of the distal osteotomy, representing the ***sliding error*** of fibula harvesting.

This is represented in **Figure 5A. Figure 5A** shows fibula distal osteotomy accuracy analyses. The distal end of fibula after harvest **310** is aligned with fibula in pre-operative virtual surgical planning **312** along the long axis **420** of the fibula. O (point **418)** is center of the planned osteotomy plane **412. O'** (point **416)** is center of the actual osteotomy plane **410.** O-O': Axial deviation (mm) of the osteotomy plane. **α**: Angle deviation (degrees) of the osteotomy plane.

The distance between the intersection points **416** and **418** generated by the real and planned osteotomy planes **410** and **412** was measured as the axial deviation of the distal osteotomy, representing the ***sliding error*** of fibula harvesting.

The device typically provides minimal sliding errors of the osteotomy guide when axial deviation, measured as the distance between intersection points generated by the real and planned osteotomy planes, is between 0 mm and about 8 mm, between about 0.5 mm and about 8 mm, between about 0.5 mm and about 7 mm, between about 0.5 mm and about 6 mm, between about 0.5 mm and about 5 mm, between about 0.5 mm and about 4 mm, between about 1 mm and about 4 mm, or about 4 mm.

When compared to osteotomies performed without the use of the device, the device typically provides minimal sliding errors of the osteotomy guide when axial deviation, measured as the distance between intersection points generated by the real and planned osteotomy planes, is between 1.5 times and 15 times less than the distance from osteotomies performed without the use of the device. The distance with the use of the device may be between about 1.5 times and about 15 times, between about 1.5 times and about 12 times, between about 1.5 times and about 10 times, between about 1.5 times and about 8 times, or between about 1.5 times and about 5 times less than the distance without the use of the device.

The distance may be a distance obtained from one patient or an average distance obtained from two or more patients.

### b. Minimizing Rotational Errors

To calculate the rotating errors, the intersection points are established on the long axis of the fibula. The intersection points are formed by the real and planned osteotomy planes intersecting the long axis of the fibula. The angulation between the real osteotomy plane and the planned osteotomy plane is the deviation in angulation of the osteotomy cut, representing the ***rotational error*** of distal fibula osteotomy.

This is represented in **Figure 5A****.** The model for the post-operative fibula after harvest **310** is aligned with fibula in pre-operative virtual surgical planning **312** along the long axis **420** of the fibula using the best fit calculation embedded in the program. The real osteotomy plane **410** was taken as the best plane that fit the osteotomy end of the distal remaining fibula. The angulation between this plane **410** and the planned osteotomy plane **412** was measured as the deviation in angulation of the osteotomy cut, representing the ***rotational error*** of distal fibula osteotomy.

The device typically provides minimal rotational errors of the osteotomy guide when angle deviation, measured as angulation between real osteotomy plane and planned osteotomy plane, is between 0 degrees and about 15 degrees, between about 0.5 degrees and about 15 degrees, between about 1 degree and about 14 degrees, between about 2 degrees and about 13 degrees, between about 2 degrees and about 12 degrees, between about 2 degrees and about 10 degrees, or about 8 degrees, about 7 degrees, about 6 degrees, about 5 degrees, about 4 degrees, about 3 degrees, about 2 degrees, about 1 degree, or about 0.5 degrees.

When compared to osteotomies performed without the use of the device, the device typically provides minimal rotational errors of the osteotomy guide when angle deviation, measured as angulation between real osteotomy plane and planned osteotomy plane, is between 1.5 time and 15 times less than the angle deviation from osteotomies performed without the use of the device. The angulation with the use of the device may be between about 1.5 times and about 15 times, between about 1.5 times and about 12 times, between about 1.5 times and about 10 times, between about 1.5 times and about 8 times, or between about 1.5 times and about 5 times less than the angulation without the use of the device.

The angle value may be a value obtained from one patient or an average value obtained from two or more patients.

### 2. Increasing Accuracy in Jaw Reconstruction with Implants

The accuracy of the jaw reconstruction surgery with or without implants may be measured by measuring platform deviation, apex deviation, and/or angle deviation between center points, apexes, and long axes angulations of the pre-operative and post-operative segments. The increased accuracy of the jaw reconstruction surgeries performed with the use of the malleolus cap may be shown by comparing the platform deviation, apex deviation, and/or angle deviation of jaw reconstructions conducted with the devices using the malleolus cap to those jaw reconstructions conducted without the use of the malleolus cap.

The accuracy measurement reference points are shown in **Figures 5B** and **5C****.** **Figure 5B** shows reconstruction segment accuracy analyses. C (point **428)** is center of the planned fibula segment **322.** C' (point **426)** is center of the actual fibula segment **320.** C-C': Center point deviation (mm) of the fibula segment. **β**: Angle deviation of the fibula segment. Absolute distance deviation represented in the color map. The angle deviation of long axes **422** and **424** of the actual fibula segment **320** and the planned fibula segment **322,** respectively.

The post-operative reconstructed jaw is typically superimposed with the pre-operative plan with the best fit of the non-operated part of native mandible. The absolute distance deviation between the surfaces of post-operative reconstruction segment **320** and the pre-operative plan **322** may be calculated using the "compare item" function embedded in the program (3-Matic 13.0) and represented by a hot map. The distance between two center points **426** and **428** is typically measured to represent the spatial deviation. The long axis of each segment **(422** and **424)** is typically generated and compared to measure the angle deviation from the plan.

**Figure 5C** shows implant accuracy analyses. Cylinder **334:** Implant position in the virtual surgical plan. Cylinder **332:** Actual implant position in the postoperative CT scan. P (point **444)** is the center point of implant platform in the virtual surgical plan. P'(point **442)** is the center point of implant platform in the actual implant. A (point **454)** is the apex of the implant in the virtual surgical plan. A' (point **452)** is the apex of actual implant placed. P-P': Deviation in implant platform position (mm). A-A': Deviation in implant apex position (mm). **γ**: Angle deviation of long axes **432** and **434** of implants.

Similar to the published methodology (Zhu, et al, *Clinical Implant Dentistry and Related Research,* Nov. 12 (2020), incorporated herein by reference), the actual location of implants inserted may be represented as the 3D models built from the post-operative CT scan, as shown in **Figure 5C****.** Long axes **432** and **434** of the actual dental implant **332** and the planned dental implant **334** are compared to demonstrate the angle deviation (y) in implant insertion. The intersection points **(442, 452)** of the long axis with the top and bottom of the dental implant are taken as the center of the platform and the implant apex. Deviation in platform and apex locations is measured to assess the accuracy of implant location.

### a. Accurate implant placement

### i. Platform deviation

Typically, the device provides accurate placement of an implant in position in a jaw when implant platform deviation, measured as a difference between center point of implant platform in the virtual surgical plan and center point of implant platform in the actual implant, is between about 0 mm and about 3 mm, between about 0 mm and about 2.5 mm, between about 0 mm and about 2 mm, between about 0 mm and about 1.5 mm, such as about 1.5 mm, about 1 mm, or about 0.5 mm.

When compared to implant placement without the use of the device, the device typically provides an accurate implant placement when implant platform deviation, measured as a difference between center point of implant platform in the virtual surgical plan and center point of implant platform in the actual implant, is between 1.5 time and 15 times less than the platform deviation from implant placements without the use of the device. The platform deviation with the use of the device may be between about 1.5 times and about 15 times, between about 1.5 times and about 12 times, between about 1.5 times and about 10 times, between about 1.5 times and about 8 times, or between about 1.5 times and about 5 times less than the platform deviation without the use of the device.

The deviation distance may be a distance obtained from one patient or an average deviation distance obtained from two or more patients.

### ii. Apex deviation

Typically, the device provides accurate placement of an implant in position in a jaw when the apex deviation, measured as a difference between apex of the implant in the virtual surgical plan and apex of actual implant placed, is between about 0 mm and about 3.5 mm, between about 0 mm and about 3 mm, between about 0 mm and about 2.5 mm, between about 0 mm and about 2 mm, between about 0 mm and about 1.5 mm, such as about 1.5 mm, about 1 mm, or about 0.5 mm.

When compared to implant placement without the use of the device, the device typically provides accurate implant placement when apex deviation, measured as a difference between apex of the implant in the virtual surgical plan and apex of actual implant placed, is between 1.5 time and 15 times less than the apex deviation from implant placements without the use of the device. The apex deviation with the use of the device may be between about 1.5 times and about 15 times, between about 1.5 times and about 12 times, between about 1.5 times and about 10 times, between about 1.5 times and about 8 times, or between about 1.5 times and about 5 times less than the apex deviation without the use of the device.

The deviation distance may be a distance obtained from one patient or an average deviation distance obtained from two or more patients.

### iii. Angle deviation

Typically, the device provides accurate placement of an implant in position in a jaw when the angle deviation, measured as angle deviation of long axes of actual implant position and implant position in the virtual surgical plan, is between about 0 degrees and about 8 degrees, between about 0.5 degrees and about 8 degrees, between about 0.5 degree and about 7 degrees, between about 1 degrees and about 7 degrees, between about 1 degrees and about 6 degrees, or about 8 degrees, about 7 degrees, about 6 degrees, about 5 degrees, about 4 degrees, about 3 degrees, about 2 degrees, about 1 degree, or about 0.5 degrees.

When compared to implant placement without the use of the device, the device typically provides accurate implant placement when the angle deviation, measured as angle deviation of long axes of actual implant position and implant position in the virtual surgical plan, is between 1.5 time and 15 times less than the angle deviation from implant placements without the use of the device. The angle deviation with the use of the device may be between about 1.5 times and about 15 times, between about 1.5 times and about 12 times, between about 1.5 times and about 10 times, between about 1.5 times and about 8 times, or between about 1.5 times and about 5 times less than the angle deviation without the use of the device.

The angle value may be a value obtained from one patient or an average value obtained from two or more patients.

### V. Kits

The caps, guides, plates, and devices may be packaged together in any suitable combination as a kit useful for performing, or aiding in the performance of, the disclosed method. It is useful if the kit components in a given kit are designed and adapted for use together in the disclosed method.

For example, disclosed are kits with one or more devices containing a malleolus cap. The kits may include devices with pre-sized malleolus caps and harvesting guides. The kits may include malleolus caps of different sizes based on an average lateral malleolus size in the population and may be in three available sizes, such as small, medium, and large. The kits may include devices with a snap connection between the connecting bar and the harvesting guide. One device may utilize numerous different harvesting guides, each of which may be snapped onto to the connecting bar of the device. The kits may include instructions use.

### Examples

Described are patient-specific 3D-printed malleolus caps and harvest devices with malleolus caps for fibula flap harvesting. The caps were used in a comparative study to evaluate their effectiveness in enhancing the accuracy of fibula osteotomy, jaw reconstruction, and simultaneous dental implant placement. The surgeries using the patient-specific 3D-printed malleolus caps produced statistically significantly greater accuracy in jaw reconstruction and implant placement versus the surgeries without the use of the malleolus caps.

### Example 1. A comparative study of using conventional measuring methods versus patient-specific malleolus caps on the accuracy of computer-assisted jaw reconstruction surgeries

### Materials and Methods

### Study Design

This was a two-arm clinical comparative study to evaluate the effectiveness of the malleolus cap design. The pre-surgical treatment plan, virtual surgery design, and surgical procedures were standardized in both arms. The single independent variable was whether a 3D-printed patient-specific fibula malleolus cap was applied to accurately locate the harvest guide intra-operatively.

### Patient Recruitment

Patient selection criteria were consistent for both groups under the 3DJP16 clinical study (ClinicalTrials.gov Identifier: NCT03057223). Briefly, patients with oral and maxillofacial benign or malignant tumors or osteoradionecrosis who needed jaw resection and fibula flap reconstruction with 3D-printed patient-specific Titanium plates were recruited. All patients were operated by the same chief surgeon in a single center. Ten consecutive patients were prospectively recruited to the study group from Jun 2020 to Dec 2020, and ten consecutive patients operated from June 2019 to Jun 2020 were retrospectively recruited as the historical control group. This study was approved by the institutional review board of the University of Hong Kong Hospital Authority Hong Kong West Cluster (UW 16-315) with the informed consent signed. The details of surgery dates are presented in Table 3.

### Pre-operative Virtual Surgical Planning

The workflow of the team in computer-assisted jaw reconstruction with 3D-printed patient-specific titanium implants was reported by Yang et al 2020. (Yang et al., Int. J. Oral Maxillofac. Surg., 49(1):13-21 (2020)). CT data were acquired and segmented to construct the 3D model of the donor fibula and the recipient jaw. Virtual reconstructive surgeries were conducted using ProPlan CMF 2.0 software (Materialise, Leuven, Belgium). Positions of simultaneous dental implants were determined in the prosthetically driven approach. Patient-specific fibula harvesting guides were designed using 3-Matic 13.0 (Materialise).

### Design of the Malleolus Cap

The lateral malleolus is a tilted pyramid structure with the most prominent point located posterior-inferiorly, which makes its lateral surface slant medial-anteriorly. As illustrated in **Figures 3A-3D****,** the lateral malleolus cap was designed as a 3 mm thick cap that fit the surface morphology of the specific patient. The inner surface of the cap was relieved by 0.5 mm for the skin covering the bony malleolus. A distal stopper **364** of 5 mm in length was added inferior to the posterior-inferior end of the fibula to prevent the axial sliding error. The cap was connected to the routine harvest guide using 1 cm diameter rigid connecting bars. The surgical guides were printed with ISO-certified bio-compatible autoclavable MED610 resin (Stratasys Ltd., United States) or NextDent SG (Vertex Dental, The Netherlands).

### Surgical Techniques

During the surgery, the 3D printed patient-specific cutting guides (guides used in head and neck for tumor resection) were fitted to the tumor resection sites, and the osteotomies were made according to the virtual surgical plan **(****Figures 2A** and **2B****).**

In the study group, the malleolus cap was fitted onto the lateral malleolus with the manual pressure in a medial-posterior direction to locate the cap anterior-posteriorly. Then the cap was pushed superiorly until the distal stopper tightly engaged the inferior end of the malleolus. When the malleolus cap was accurately located, the osteotomy guide fitted onto the fibula bone surface automatically. In this way, the location of the fibula harvest guide (also referred to as surgery device, represented by the combination of the osteotomy guide and malleolus cap was controlled in all three dimensions.

In the control group, lateral malleolus was marked on the skin by palpation. After exposure of the fibula, the harvest guide was fitted to the estimated location by measuring the distance from the lateral malleolus skin marking to the osteotomy site according to the virtual surgical plan.

Distal fibula osteotomies were performed with the fibula harvest guides in both groups. Simultaneous dental implants were placed into the fibula before segmentation. Fibula flap segments were transferred to repair the defect and fixed to the remaining jaw with 3D-printed patient-specific titanium plates **(****Figures 4A-4D****).**

### Outcomes Assessment

Spiral CT of the lower limbs and reconstructed jaws were acquired postoperatively. Based on the data of CT scan, three-dimensional models of the distal end of the remaining fibula, reconstructed jaws, and simultaneous dental implants were built using ProPlan CMF 2.0 software (Materialise, Leuven, Belgium). The 3D models were imported to 3-Matic 13.0 (Materialise) for comparison of the corresponding items with the pre-operative plan. The references used for analyses are illustrated in **Figures 5A-5C****.**

### Fibula osteotomy accuracy analyses

The post-operative fibula model **310** was superimposed with the pre-operative fibula **312** using the best fit calculation embedded in the program. The real osteotomy plane **(410)** was taken as the best plane that fit the osteotomy end of the distal remaining fibula. The angulation between this plane **(410)** and the planned osteotomy plane **(412)** was measured as the deviation in angulation of the osteotomy cut, representing the ***rotational error*** of distal fibula osteotomy. The long axis of the fibula **(420)** was created and the intersection points **416** and **418** along the long axis **420** of the two osteotomy planes were taken to mark the location of the osteotomy planes **410** and **412,** respectively. The distance between the intersection points **416** and **418** generated by the real and planned osteotomy planes **410** and **412** was measured as the axial deviation of the distal osteotomy, representing the ***sliding error*** of fibula harvesting.

### Mandible reconstruction analyses

As described in the previous study (Yang et al., Ann Surg Oncol., 28:363-375 (2021), published online 2020), the post-operative reconstructed jaw was superimposed with the pre-operative plan with the best fit of the non-operated part of native mandible. The absolute distance deviation between the surfaces of the actual fibula segment **320** and the planned fibula segment **322** was calculated using the "compare item" function embedded in the program (3-Matic 13.0) and represented by a hot map. The distance between two center points **426** and **428** was measured to represent the spatial deviation. The long axis of each segment **(422** and **424)** was generated and compared to measure the angle deviation from the plan.

### Simultaneous dental implant accuracy analyses

Similar to the published methodology (Zhu, et al, Clinical Implant Dentistry and Related Research, Nov. 12 (2020)), the actual location of implants inserted was represented as the 3D models built from the post-operative CT scan. Long axes **432** and **434** of the actual implant position **332** and the implant position in the virtual surgical plan **334** were compared to demonstrate the angle deviation in implant insertion. The intersection points **(442, 452)** of the long axis with the top and bottom of the dental implant were taken as the center of the platform and the implant apex. Deviation in platform and apex locations was measured to assess the accuracy of implant location.

### Statistical Analysis

All statistical analyses were performed using IBM SPSS Statistics Version 25. Categorical data were presented as counts with proportions and compared using Chi-square test or Fisher's exact test where appropriate. The normality of continuous data was tested using Shapiro-Wilk's test (p>0.05). Descriptive statistical analyses were performed with results presented by mean with standard deviation (SD) for normally distributed data and medians with interquartile range (IQR) for skewed data. Independent-samples t-test and Mann-Whitney *U* test were used to compare the difference between the two groups for normally distributed data and skewed data respectively. All tests and reported p values were two-sided. A *p*-value of less than 0.05 was considered statistically significant.

### Results

### Patient Demographics

Twenty patients who underwent jaw reconstructions with fibula free flaps and 3D-printed patient-specific Titanium plates were included in the study with ten patients in each arm. All twenty fibula free flaps survived. The postoperative follow-up rate was 100%. A total of 13 and 18 simultaneous dental implants were inserted in the study and control groups respectively. The demographic data and reconstruction characteristics are presented in Table 1. No significant difference was detected between the two groups.

**Table 1. Patient Demographics and Reconstruction Characteristics.**

| | | | Study (n=10) | Control (n=10) | *p* value |
|---|---|---|---|---|---|
| Gender | | | | | |
| | Male | | 6 | 4 | 0.66 |
| | Female | | 4 | 6 | |
| Age (mean) | | | 53 | 60 | 0.44 |
| Diagnosis | | | | | 0.80 |
| | SCC | | 5 | 7 | |
| | Other malignancy | | 1 | 1 | |
| | | Benign jaw lesions | 2 | 2 | |
| | | Osteoradionecrosis | 2 | 0 | |
| Reconstruction Site | | | | | |
| | Maxilla | | 2 | 2 | 1.00 |
| | Mandible | | 8 | 8 | |
| Staging | | | | | |
| | pT1/2 | | 0 | 3 | 0.31 |
| | pT3/4 | | 6 | 5 | |
| | NA | | 4 | 2 | |
| Fibula Segments (mean) | | | 2 | 2 | 0.73 |
| Implants (total) | | | 13 | 18 | 0.42 |

### Outcomes Analyses

The accuracy of distal osteotomies of the fibulas, reconstruction segments, and implants was analyzed for all twenty cases and compared between the study and the control groups. The post-operative measurements for accuracy analyses were performed by two independent assessors blinded from the grouping. The inter-assessor agreement was good to excellent. The average values of the two assessors were taken for the final analyses. The results are presented in Table 2 and **Figures 6A-6H****.**

### Fibula Donor Site Osteotomy Accuracy Analyses

The accuracy of the distal osteotomy of the fibula increased significantly in the malleolus cap group. The axial deviation in location of the osteotomy plane from the virtual surgical plan decreased from 9.5 ± 6.3 mm to 4.1 ± 2.7 mm (mean difference = -5.5 mm, 95% CI = -0.9 to -10.0, p=0.02, **Figure 6B****).** The deviation of the distal osteotomy angle decreased from 25.3° ± 13.1° (mean difference = -16.6 °, 95% CI = -6.9 to -26.4, p<0.01, **Figure 6A****).**

### Reconstruction Segment Analyses

Three-dimensionally printed patient-specific Titanium plates were used in both groups. No significant difference in the accuracy of reconstruction segments was detected in terms of absolute distance deviation, reconstruction segment angle deviation, or reconstruction segment center point deviation **(****Figures 6C-6E****).**

### Implant Accuracy Analyses

There was a significant improvement in the accuracy of simultaneous dental implants in the malleolus cap group. The platform deviation from the virtual surgical plan decreased from 3.2 ± 1.4 mm to 1.3 ± 0.8 mm (mean difference = -1.8 mm, 95% CI = -1.1 to -2.6, p<0.01, **Figure 6G****).** The deviation of apical point of the implants decreased from 3.8 ± 1.3 mm to 1.5 ± 0.8 mm (mean difference = -2.2 mm, 95% CI = -1.4 to -3.1, p<0.01, **Figure 6H****).** The angle deviation reduced to 4.6 ± 1.7° in the study group compared to 11.3 ± 7.3° in the control group (mean difference = -6.8°, 95% CI = -3.1° to -10.5°, p=0.01, **Figure 6F****).**

**Table 2. Accuracy Analyses Results**

| | | Study (n=10) | Control (n=10) | p-value | Mean Differ ence | 95% Confidence Interval |
|---|---|---|---|---|---|---|
| Fibula Donor Site Analyses | | | | | | |
| | Fibula Osteotomy | | | | | -0.9 to - |
| | - Axial Deviation (mm) | 4.1 ± 2.7 | 9.5 ± 6.3 | 0.02* | -5.5 | 10.0 |
| | - Angle Deviation (degrees) | 8.7 ± 5.0 | 25.3 ± 13.1 | <0.01** | -16.6 | -6.9 to - 26.4 |
| Reconstruction Segment Analyses | | | | | | |
| | Absolute Distance Deviation | 1.5 ± 0.8 | 1.9 ± 0.7 | 0.23 | | |
| | Angle Deviation | 5.2 ± 2.5 | 5.8 ± 2.8 | 0.63 | | |
| | Center Points | 2.1 ± 1.8 | 3.9 ± 1.3 | 0.44 | | |
| Implant Analyses | | | | | | |
| | Platform Deviation | 1.3 ± 0.8 | 3.2 ± 1.4 | <0.01** | -1.8 | -1.1 to -2.6 |
| | Apex Deviation | 1.5 ± 0.8 | 3.8 ± 1.3 | <0.01** | -2.2 | -1.4 to -3.1 |
| | Angle Deviation | 4.6 ± 1.7 | 11.3 ± 7.3 | 0.01* | -6.8 | -3.1 to - 10.5 |

This is the first clinical study providing reduced sliding and rotational errors of fibula flap harvesting. The developed fibula malleolus cap demonstrated the effectiveness in accurately guiding the location and angulation during fibula flap harvesting in a clinical comparative study.

The difference in geometric shape of cross-sectional anatomy of fibula has been an ignored issue in fibula flap jaw reconstruction so far. When simultaneous dental implantation is planned, the changes in cross-sectional shapes (such as oval, triangle, quadrilateral, and pentagonal, **Figures 1A-1D****)** can make a significant difference in dental implant position and angulation. Traditionally, when harvesting the fibula free flap, the location of the fibula cutting guide was determined by the measurement from the lateral malleolus. This leads to sliding and rotational errors as shown in **Figures 1E** and **1F****.** In this study, the sliding error in the control group was as large as 9.5 mm. With the application of the malleolus cap design, the error was reduced by more than half to 4.1 mm. This sliding error will lead to different shapes of the fibula harvested compared to the preoperative plan. The rotational error was also a concern especially when the fibula was covered with the periosteum and a thin cuff of muscle when the fibula cutting guide was fitted. The results showed the malleolus cap could significantly reduce the rotational error and improve the accuracy of cutting angle from 25.3 to 8.7 degrees.

Interestingly, the results showed no significant difference in the accuracy of reconstructive fibula segments with or without the use of malleolus cap. The average deviation of the surface location, center point, and angulation in this study was comparable to the studies by Schepers et al., J Craniomaxillofac Surg., 43:649-57 (2015) and De Maesschalck et al., Eur Arch Otorhinolaryngol., 274:517-526 (2017). The accuracy of fibula segments in reconstructive mandible is mostly determined by the mandible osteotomy guides and 3D-printed patient-specific Titanium plate (Yang et al., Ann Surg Oncol., 28:363-375 (2021), published online 2020). The sliding and rotational errors in fibula harvesting might not be clinically significant if simultaneous dental implants were not planned. Although currently there has been no widely accepted criteria for minimal clinically important difference (MCID) in jaw reconstruction, MCID will be different whether dental implants rehabilitation is to be performed or not. The application of simultaneous dental implants has further pushed the front of functional jaw reconstruction with dental rehabilitation. When simultaneous dental implants were planned, it required high accuracy of the fibula harvest guides to insert the implants into the designated position and angulation.

The accuracy of simultaneous implants was significantly increased in the fibula malleolus cap group. Literature showed that the designs of implant positioning guides and methods of accuracy analyses varied, which made the comparison between studies difficult. Shepers 2016 reported a center deviation of 5.5 mm and angle deviation of 6.1° in their group of simultaneous dental implants in jaw reconstruction (Shepers et al., J Craniomaxillofac Surg., 44(4):392-399 (2016)). With the application of the malleolus cap in the present study, implant platform and apex deviations were reduced to 1.3 mm and 1.5 mm respectively, with an angular deviation of 4.6 degrees. This accuracy level approached what could be achieved with the conventional guided dental implant placement directly into the native maxilla and mandible. Meta-analysis by Tahmaseb et al. (Tahmaseb et al., Clin Oral Implants Res., 29(Suppl 16):416-435 (2016) reported a deviation of 1.2 mm at the entry point and 1.4 mm at the apex with an angle deviation of 3.5 degrees. This proved that with careful pre-surgical planning and good intra-operative execution, the accuracy of simultaneous dental implants in fibula free flaps can be comparable with the dental implants placed directly into the native jaws.

There are certain limitations in this study. A recent historical control group was adopted in the study. There was no randomization between the two groups. However, all the surgeries were performed by the same chief surgeon with the same design of surgical guides and patient-specific Titanium plates except the difference of malleolus cap, and the difference of median date of surgery between two groups was only 6.5 months, thus reducing the bias to a minimum.

This is the first study assessing the accuracy of the fibula harvest guide in guiding the location and angulation of the fibula osteotomy. The use of the developed fibula malleolus cap significantly increased the accuracy of the fibula osteotomy thus making the dental prosthetic rehabilitation with simultaneous dental implants more precise, approaching the similar accuracy level of the guided implant surgeries in native maxilla and mandible.

**Table 3. Details of patients' diagnoses, lesion sites, surgery dates (shown in date/month/year), fibula segments, and the number of implants.**

| Group | Gender | Age | Diagnosis | Lesion Site | Staging | Surgery Date | Fibula Segments | Implants |
|---|---|---|---|---|---|---|---|---|
| Study 1 | M | 58 | SCC | Right FOM | pT4aN0 | 26/6/2020 | 3 | 0 |
| Study 2 | F | 37 | Mucoepidermoid Ca | Right maxilla | pT4aN0 | 20/10/2020 | 1 | 2 |
| Study 3 | F | 73 | SCC | Right mandible | pT4aN0 | 18/6/2020 | 2 | 0 |
| Study 4 | M | 21 | Ameloblastoma | Right mandible | NA | 23/7/2020 | 2 | 4 |
| Study 5 | F | 76 | ORN | Left mandible | NA | 5/11/2020 | 1 | 3 |
| Study 6 | M | 68 | SCC | Left mandible | rT4b | 8/9/2020 | 2 | 0 |
| Study 7 | M | 32 | Ossifying fibroma | Anterior mandible | NA | 6/8/2020 | 3 | 4 |
| Study 8 | M | 32 | SCC | Right palate | pT4bN0 | 11/8/2020 | 3 | 0 |
| Study 9 | M | 66 | SCC | Left mandible | mpT4a | 12/11/2020 | 2 | 0 |
| Study 10 | F | 65 | ORN | Left mandible | NA | 1/12/2020 | 1 | 0 |
| Control 1 | F | 76 | SCC | Left mandible | pT2N0 | 8/11/2019 | 2 | 3 |
| Control 2 | M | 85 | SCC | Right mandible | pT4N0 | 28/11/2019 | 3 | 0 |
| Control 3 | F | 60 | Ameloblastoma | Left maxilla | NA | 27/2/2020 | 2 | 3 |
| Control 4 | F | 22 | Chondrosarcoma | Left mandible | pT1N0M0G3 (IIa) | 23/1/2020 | 1 | 0 |
| Control 5 | F | 30 | Ameloblastoma | Right mandible | NA | 22/4/2020 | 2 | 2 |
| Control 6 | M | 40 | SCC | Left mandible | pT4aN0 | 4/6/2020 | 3 | 5 |
| Control 7 | F | 66 | SCC | Left maxilla | pT2N0 | 29/4/2020 | 1 | 3 |
| Control 8 | M | 66 | SCC | Right retromolar trigone | pT4aN0 | 8/4/2020 | 2 | 0 |
| Control 9 | M | 82 | SCC | Right mandible | pT3N0 | 15/8/2019 | 2 | 2 |
| Control 10 | F | 75 | SCC | Left cheek | pT4aN2a | 19/6/2019 | 2 | 0 |

## Claims

1. A surgical device (300) comprising
a patient-specific malleolus cap (360) having a surface morphology or contour modelled according to the morphology of a lateral malleolus of the intended patient, and an osteotomy guide (350),
wherein the surgical device further comprises a connecting bar;
wherein the patient-specific malleolus cap is detachably or non-detachably connected to the osteotomy guide via the connecting bar (220); and wherein the osteotomy guide is a fibula cutting guide or a fibula harvest guide.

2. The surgical device of claim 1, wherein the patient-specific malleolus cap comprises an inner cap surface and an outer cap surface, wherein the outer cap surface contacts one end of the connecting bar.

3. The surgical device of claim 1, wherein the patient-specific malleolus cap comprises an inner cap surface and an outer cap surface, wherein the inner cap surface comprises morphology modelled to fit the surface morphology of the patient's lateral malleolus.

4. The surgical device of claim 1, wherein the patient-specific malleolus cap comprises a longitudinal axis (A) substantially parallel to longitudinal axis of the connecting bar and longitudinal axis of the osteotomy guide.

5. The surgical device of claim 4, wherein longitudinal axes of the malleolus cap, the connecting bar, and the osteotomy guide are offset relative to one another.

6. The surgical device of claim 5, wherein longitudinal axes of the malleolus cap, the connecting bar, and the osteotomy guide are offset relative to one another by a distance between about 0.2 mm and 50 mm.

7. The surgical device of claim 1, wherein the connecting bar contacts the patient-specific malleolus cap and the osteotomy guide via connecting rods contacting the superior surface of the patient-specific malleolus cap and the osteotomy guide at an obtuse, right, or acute angle.

8. The surgical device of claim 1, wherein the osteotomy guide is a patient-specific fibula cutting guide or a patient-specific fibula harvest guide.

9. The surgical device of claim 1, wherein patient-specific malleolus cap, the osteotomy guide, and the connecting bar are formed from biocompatible, autoclavable resin, polymer, metal, or metal alloy.

10. The surgical device of claim 1, wherein the device is made by a method selected from the group consisting of stereolithography, soft lithography, laser machining, laser cutting, micromachining, micromilling, curing, bonding, three-dimensional printing, molding, micromolding, and setting.

11. The surgical device of claim 1, wherein the device is configured for use in reconstruction surgery of head and neck bone defects, for use in jaw reconstruction surgery, or for use in jaw reconstruction surgery with dental implant placement.

12. A kit comprising the surgical device of claim 1.

## Patentansprüche

1. Chirurgische Vorrichtung (300), umfassend
eine patientenspezifische Knöchelkappe (360) mit einer Oberflächenmorphologie oder Kontur, die gemäß der Morphologie eines lateralen Knöchels des beabsichtigten Patienten modelliert ist, und eine Osteotomieführung (350),
wobei die chirurgische Vorrichtung ferner eine Verbindungsstange umfasst;
wobei die patientenspezifische Knöchelkappe über die Verbindungsstange (220) abnehmbar oder nicht abnehmbar mit der Osteotomieführung verbunden ist; und wobei die Osteotomieführung eine Wadenbein-Schnittführung oder eine Wadenbein-Entnahmeführung ist.

2. Chirurgische Vorrichtung nach Anspruch 1, wobei die patientenspezifische Knöchelkappe eine innere Kappenoberfläche und eine äußere Kappenoberfläche umfasst, wobei die äußere Kappenoberfläche ein Ende der Verbindungsstange berührt.

3. Chirurgische Vorrichtung nach Anspruch 1, wobei die patientenspezifische Knöchelkappe eine innere Kappenoberfläche und eine äußere Kappenoberfläche umfasst, wobei die innere Kappenoberfläche eine Morphologie umfasst, die so modelliert ist, dass sie der Oberflächenmorphologie des lateralen Knöchels des Patienten entspricht.

4. Chirurgische Vorrichtung nach Anspruch 1, wobei die patientenspezifische Knöchelkappe eine Längsachse (A) umfasst, die im Wesentlichen parallel zu der Längsachse der Verbindungsstange und der Längsachse der Osteotomieführung ist.

5. Chirurgische Vorrichtung nach Anspruch 4, wobei Längsachsen der Knöchelkappe, der Verbindungsstange und der Osteotomieführung relativ zueinander versetzt sind.

6. Chirurgische Vorrichtung nach Anspruch 5, wobei die Längsachsen der Knöchelkappe, der Verbindungsstange und der Osteotomieführung um einen Abstand zwischen etwa 0,2 mm und 50 mm relativ zueinander versetzt sind.

7. Chirurgische Vorrichtung nach Anspruch 1, wobei die Verbindungsstange die patientenspezifische Knöchelkappe und die Osteotomieführung über Verbindungsstäbe berührt, welche die obere Oberfläche der patientenspezifischen Knöchelkappe und der Osteotomieführung in einem stumpfen, rechten oder spitzen Winkel berühren.

8. Chirurgische Vorrichtung nach Anspruch 1, wobei die Osteotomieführung eine patientenspezifische Wadenbein-Schnittführung oder eine patientenspezifische Wadenbein-Entnahmeführung ist.

9. Chirurgische Vorrichtung nach Anspruch 1, wobei die patientenspezifische Knöchelkappe, die Osteotomieführung und die Verbindungsstange aus biokompatiblem, autoklavierbarem Harz, Polymer, Metall oder einer Metalllegierung gebildet sind.

10. Chirurgische Vorrichtung nach Anspruch 1, wobei die Vorrichtung durch ein Verfahren hergestellt ist, das aus der Gruppe ausgewählt ist, die aus Stereolithographie, weicher Lithographie, Laserbearbeitung, Laserschneiden, Mikrobearbeitung, Mikrofräsen, Härten, Kleben, dreidimensionales Drucken, Formen, Mikroformen und Setzen besteht.

11. Chirurgische Vorrichtung nach Anspruch 1, wobei die Vorrichtung zur Verwendung bei der Rekonstruktionschirurgie von Kopf- und Halsknochendefekten, zur Verwendung bei der Kieferrekonstruktionschirurgie oder zur Verwendung bei der Kieferrekonstruktionschirurgie mit Zahnimplantatplatzierung konfiguriert ist.

12. Kit, umfassend die chirurgische Vorrichtung nach Anspruch 1.

## Revendications

1. Dispositif chirurgical (300) comprenant :
une coiffe de malléole (360) spécifique au patient comportant une morphologie de surface ou un contour modélisé selon la morphologie d'une malléole latérale du patient visé, et
un guide d'ostéotomie (350),
ledit dispositif chirurgical comprenant en outre une barre de raccordement ;
ladite coiffe de malléole spécifique au patient étant raccordée de manière amovible ou non amovible au guide d'ostéotomie par l'intermédiaire de la barre de raccordement (220) ; et
ledit guide d'ostéotomie étant un guide de découpe de péroné ou un guide de prélèvement de péroné.

2. Dispositif chirurgical selon la revendication 1, ladite coiffe de malléole spécifique au patient comprenant une surface de coiffe interne et une surface de coiffe externe, la surface de coiffe externe étant en contact avec une extrémité de la barre de raccordement.

3. Dispositif chirurgical selon la revendication 1, ladite coiffe de malléole spécifique au patient comprenant une surface de coiffe interne et une surface de coiffe externe, ladite surface de coiffe interne comprenant une morphologie modélisée destinée à s'adapter à la morphologie de surface de la malléole latérale du patient.

4. Dispositif chirurgical selon la revendication 1, ladite coiffe de malléole spécifique au patient comprenant un axe longitudinal (A) sensiblement parallèle à l'axe longitudinal de la barre de raccordement et à l'axe longitudinal du guide d'ostéotomie.

5. Dispositif chirurgical selon la revendication 4, lesdits axes longitudinaux de la coiffe de malléole, de la barre de raccordement et du guide d'ostéotomie étant décalés les uns par rapport aux autres.

6. Dispositif chirurgical selon la revendication 5, lesdits axes longitudinaux de la coiffe de malléole, de la barre de raccordement et du guide d'ostéotomie étant décalés les uns par rapport aux autres d'une distance comprise entre environ 0,2 mm et 50 mm.

7. Dispositif chirurgical selon la revendication 1, ladite barre de raccordement étant en contact avec la coiffe de malléole spécifique au patient et le guide d'ostéotomie par l'intermédiaire de tiges de raccordement en contact avec la surface supérieure de la coiffe de malléole spécifique au patient et le guide d'ostéotomie suivant un angle obtus, droit ou aigu.

8. Dispositif chirurgical selon la revendication 1, ledit guide d'ostéotomie étant un guide de découpe de péroné spécifique au patient ou un guide de prélèvement de péroné spécifique au patient.

9. Dispositif chirurgical selon la revendication 1, ladite coiffe de malléole spécifique au patient, ledit guide d'ostéotomie et ladite barre de raccordement étant formés à partir d'une résine, d'un polymère, d'un métal ou d'un alliage métallique biocompatible et autoclavable.

10. Dispositif chirurgical selon la revendication 1, ledit dispositif étant fabriqué par un procédé choisi dans le groupe constitué par la stéréolithographie, la lithographie douce, l'usinage au laser, la découpe au laser, le micro-usinage, le micro-moulage, le durcissement, le collage, l'impression tridimensionnelle, le moulage, le micro-moulage et le durcissement.

11. Dispositif chirurgical selon la revendication 1, ledit dispositif étant conçu pour être utilisé dans la chirurgie de reconstruction de défauts osseux de la tête et du cou, pour être utilisé dans la chirurgie de reconstruction de la mâchoire, ou pour être utilisé dans la chirurgie de reconstruction de la mâchoire avec placement d'implant dentaire.

12. Kit comprenant le dispositif chirurgical selon la revendication 1.
